# EUROPEAN PATENT SPECIFICATION

(11) **EP 3 638 196 B1**
(45) Date of publication and mention of the grant of the patent: **13.09.2023**
(21) Application number: 18730719.4
(22) Date of filing: 07.06.2018
(51) Int. Cl.: A61K 8/34, A61K 8/41, A61Q 5/12, A61K 8/81

(54) **HAIR CONDITIONING COMPOSITION FOR IMPROVED RINSE**
HAARKONDITIONIERENDE ZUBEREITUNG
PRÉPARATION DE CONDITIONNEMENT DES CHEVEUX

(30) Priority: 15.06.2017 EP 17176250
(43) Date of publication of application: 22.04.2020
(73) Proprietor: Unilever IP Holdings B.V., 3013 AL Rotterdam (NL); Unilever Global IP Limited, Wirral, Merseyside CH62 4ZD (GB)
(72) Inventor: COAN, Lynsey, Joanne, Bebington Wirral Merseyside CH63 3JW (GB); GILES, Colin, Christopher, David, Bebington Wirral Merseyside CH63 3JW (GB); GLENDAY, Jennifer, Amy, Sheffield S8 8TD (GB); GUTIERREZ-ABAD, Raquel, Bebington Wirral Merseyside CH63 3JW (GB); LUCK, Matias, Wirral Merseyside CH62 2EY (GB)
(74) Representative: Chisem, Janet
(86) International application number: PCT/EP2018/064956
(87) International publication number: WO 2018/228899

(56) References cited:
- WO-A1-2009/153281
- WO-A1-2015/047826
- WO-A2-2006/081496
- DATABASE GNPD [Online] MINTEL; 1 April 2018 (2018-04-01), "Smooth and Serene Conditioner", XP002783624, Database accession no. 5563543
- Umbach: "Kosmetik" In: "Kosmetik", 1 January 1998 (1998-01-01), XP055393819, ISBN: 978-3-527-30875-0 pages 250-251,
- DATABASE GNPD [Online] MINTEL; 1 February 2016 (2016-02-01), "Shampoo", XP002772512, Database accession no. 3761041
- DATABASE GNPD [Online] MINTEL; 1 March 2009 (2009-03-01), "Shave Cream", XP002772513, Database accession no. 1063603
- DATABASE GNPD [Online] MINTEL; 1 November 2008 (2008-11-01), "Strawberry Lemonade In Shower Body Lotion", XP002772514, Database accession no. 1007224
- "Aculyn? 28 Rheology Modifier/Stabilizer A Very Efficient Thickener for a Wide Array of Personal Care Formulations", ROHM HAAS PERSONAL,, 1 February 2004 (2004-02-01), pages 1-16, XP007909941,

## Description

### Field of the Invention

The invention is concerned with compositions that are formulated as conditioners for the treatment of hair (typically after shampooing) and subsequent rinsing and particularly relates to an improved rinse-off hair conditioner composition that enables less water to be used during use.

### Background and Prior art

Reducing the amount of water that is used in everyday tasks and activities, such as washing of hair, reduces the energy required to process and deliver it to homes, businesses and communities. This in turn helps to reduce pollution and conserve fuel resources. Reducing the amount of water that goes to waste at home helps protect the wildlife that lives in rivers and wetlands. Thus, hair conditioner products that require reduced rinsing to remove from the hair not only save consumers time and effort but can also save ecosystems, energy and water.

Technologies are known that improve rinsing properties of products from hair or hands.

WO 13/092708 (L'Oreal) discloses a cosmetic composition, especially a hair composition, comprising at least one anionic or nonionic associative polymer, at least one fixing polymer and at least one specifically defined nonionic surfactant. A styling gel conforming to this composition is purported to have improved hold of the hairstyle over time and to be easily removed from the hands and the hair with water, without shampoo or soap.

WO 15/001071 (L'Oreal) discloses a non-colouring hair composition comprising 2 - 60 wt % of at least one anionic copolymer, water-soluble inorganic salts and one or more alkaline agents. The anionic copolymer may be an anionic associative polymer, such as are capable of associating reversibly with one another or with other molecules. Numerous benefits of these compositions are disclosed, and they are said to be particularly capable of generating an adequate foam, in terms of quality and quantity, and of giving the hair satisfactory cosmetic properties, such as sheen, softness, smoothness, disentangling and suppleness, most particularly on dry hair. Further they are purported to be rinsed out faster than conventional shampoos, to have a more pronounced treating nature and to give the hair more lightness, in particular wet hair. They don't necessarily require the addition of viscosity enhancers, can afford varied textures and are better tolerated by the scalp and the eyes. Detergent compositions used on hair which are said to provide a foaming effect and good cosmetic properties, are exemplified.

Related case WO15/001072 (L'Oreal) discloses a self-foaming non-colouring hair composition comprising from 2 to 60 wt % of one or more anionic, or non-ionic associative polymers, surfactant and propellant gas.

WO 09/153281 (Unilever) discloses a hair conditioning composition comprising hydrophobically modified anionic polymer. The inclusion of a crystalline wax as a structurant is preferred and exemplified. The polymer is said to provide better rinse-off properties and an example discloses better ease of rinse in a composition comprising the polymer compared with a similar composition without the polymer.

Related case WO 09/153280 (Unilever) discloses hair conditioning compositions comprising hydrophobically modified anionic polymer, a silicone and a fatty acid to give improved deposition of silicone. Better rinse off properties are mentioned but silicone deposition is exemplified.

Compositions that are easy to rinse do not necessarily require less water to accomplish the rinse. They may, for example, require less mechanical agitation, or even no agitation, but a longer rinse time, so less effort is required but not less water. Despite the technological advances there remains a need for conditioning compositions for use on hair, that require reduced water to rinse effectively and quickly without compromising the performance of the product as a conditioner, particularly is bestowing good conditioned wet and dry feel characteristics such as smooth wet feel and soft dry feel.

We have now surprisingly found that a hair conditioner composition that comprises a conditioning base and a hydrophobically modified anionic polymer can be used in the treatment of hair to reduce the amount of water required to rinse without reducing the conditioning benefits on the hair. We have found that when a consumer rinses conditioner from his/her hair, he/she will stop rinsing when a satisfactory constant level of smooth feel is reached (referred to herein as the "rinsed friction plateau"). The composition of the invention enables the consumer to reach his/her rinsed friction plateau sooner, thus causing him/her to stop rinsing and therefore consume less water.

### Definition of the Invention

Accordingly, there is provided a hair conditioner composition comprising
a) a conditioning base comprising
   i) from 0.01 to 10 wt %, by weight of the composition of a cationic surfactant,
   ii) from 0.01 to 10 wt %, by weight of the composition of a fatty alcohol having from 8 to 22 carbon atoms;
b) from 0.01 to 5.0 wt %, by weight of the composition of a hydrophobically modified anionic polymer, comprising hydrophobic modification that comprises an alkyl group comprising from 6 to 30 carbon atoms; and
c) water
wherein the hair conditioner composition comprises from 0 to less than 0.1 weight %, by weight of the total composition of stearamidopropyldimethylamine.

In a second aspect, the invention provides a method of saving water during a conditioning process, comprising the steps of applying to hair a composition of the first aspect and rinsing the hair with water.

In a third aspect, the invention provides a use of a hydrophobically modified anionic polymer in a hair conditioner composition, saving water during a conditioning process.

In the method of the invention, preferably, the hair is rinsed with water until the friction levels out to a plateau.

The use of the invention saves water without compromising on the performance of the product, in terms of wet conditioning and dry conditioning.

The use of the invention, is preferably to save water during a rinse step of a conditioning process where the hair is rinsed until the friction levels out to a plateau.

The amount of water required to rinse the composition form hair is reduced by the method of the invention. Thus less water is required when using a composition of the invention. Therefore, water is saved.

The method of the invention preferably saves water during the rinse step of conditioning process.

The compositions of the invention are intended for use for application to hair on the head.

### The hydrophobically modified anionic polymer

Preferably, the hydrophobically modified polymer is an acrylate or methacrylate polymer.

The hydrophobic modification comprises an alkyl group. The alkyl group comprises from C12 to C30, preferably from 16 to 28 and most preferably from 18 to 24 carbons.

A preferred polymer is sold by Rohm & Haas under the tradename Aculyn, the most preferred of which is Aculyn 28^{™}.

The polymer is present at from 0.01 to 5 wt %, more preferably from 0.02 to 0 5 wt %, even more preferably from 0.03 to 4 wt % and most preferably from 0.05 to 4 wt %, by total weight of the hair conditioner composition.

### The conditioning base

Compositions in accordance with the invention are preferably formulated as conditioners for the treatment of hair (typically after shampooing) and subsequent rinsing.

The conditioning base forms a gel phase.

### The monalkyl amine and acid

The conditioning base comprises a combination of i) a cationic surfactant and ii) a fatty alcohol.

The compositions of the invention comprises from 0 to less than 0.1 weight %, preferably less than 0.05 weight %, more preferably less than 0.001 weight %, even more preferably less than 0.0001 weight %, and most preferably 0 wt %, by weight of the total composition, of from stearamidopropyldimethylamine (TAS); preferably from 0 to less than 0.1 weight %, preferably less than 0.05 weight %, more preferably less than 0.001 weight %, even more preferably less than 0.0001 weight %, and most preferably 0 wt % by weight of the total composition of each of stearamidopropyldimethylamine and stearamidopropyl dimethylamine stearate; more preferably from 0 to less than 0.1 weight %, preferably less than 0.05 weight %, more preferably less than 0.001 weight %, even more preferably less than 0.0001 weight %, and most preferably 0 wt %, by weight of the total composition of each of stearamidopropyldimethylamine, stearamidopropyl dimethylamine stearate and stearamidoethyldiethylamine, and most preferably from 0 to less than 0.1 weight %, preferably less than 0.05 weight %, more preferably less than 0.001 weight %, even more preferably less than 0.0001 weight %, and most preferably 0 wt % from amidoamine.

### The fatty alcohol

The compositions of the invention comprise a fatty alcohol having a carbon-carbon chain length of from C₈ to C₂₂.

The combined use of fatty alcohols and cationic surfactants in conditioning compositions is preferred because this leads to the formation of a lamellar phase, in which the cationic surfactant is dispersed.

The fatty alcohol comprises from 8 to 22 carbon atoms, preferably 16 to 22. Fatty alcohols are typically compounds containing straight chain alkyl groups. Examples of suitable fatty alcohols include cetyl alcohol, stearyl alcohol and mixtures thereof. The use of these materials is also advantageous in that they contribute to the overall conditioning properties of compositions for use in the invention.

The level of fatty alcohol in conditioners for use in the invention is from 0.01 to 10%, preferably from 0.1 to 8%, more preferably from 0.2 to 7%, most preferably from 0.3 to 6% by weight of the composition. The weight ratio of cationic surfactant to fatty alcohol is suitably from 1:1 to 1:10, preferably from 1:1.5 to 1:8, optimally from 1:2 to 1:5. If the weight ratio of cationic surfactant to fatty alcohol is too high, this can lead to eye irritancy from the composition. If it is too low, it can make the hair feel squeaky for some consumers.

### The cationic surfactant

The conditioning base comprises a cationic conditioning surfactant.

Preferably, the cationic conditioning surfactant has the formula N⁺(R¹)(R²)(R³)(R⁴), wherein R¹, R², R³ and R⁴ are independently (C₁ to C₃₀) alkyl or benzyl.

Preferably, one, two or three of R¹, R², R³ and R⁴ are independently (C₄ to C₃₀) alkyl and the other R¹, R², R³ and R⁴ group or groups are (C₁-C₆) alkyl or benzyl.

More preferably, one or two of R¹, R², R³ and R⁴ are independently (C₆ to C₃₀) alkyl and the other R¹, R², R³ and R⁴ groups are (C₁-C₆) alkyl or benzyl groups. Optionally, the alkyl groups may comprise one or more ester (-OCO- or -COO-) and/or ether (-O-) linkages within the alkyl chain. Alkyl groups may optionally be substituted with one or more hydroxyl groups. Alkyl groups may be straight chain or branched and, for alkyl groups having 3 or more carbon atoms, cyclic. The alkyl groups may be saturated or may contain one or more carbon-carbon double bonds (e.g., oleyl). Alkyl groups are optionally ethoxylated on the alkyl chain with one or more ethyleneoxy groups.

Suitable cationic conditioning surfactants for use in conditioner compositions according to the invention include cetyltrimethylammonium chloride, behenyltrimethylammonium chloride, cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, dihydrogenated tallow dimethyl ammonium chloride (e.g., Arquad 2HT/75 from Akzo Nobel), cocotrimethylammonium chloride, PEG-2-oleammonium chloride and the corresponding hydroxides thereof. Further suitable cationic surfactants include those materials having the CTFA designations Quaternium-5, Quaternium-31 and Quaternium-18. Mixtures of any of the foregoing materials may also be suitable. A particularly useful cationic surfactant for use in conditioners according to the invention is cetyltrimethylammonium chloride, available commercially, for example as GENAMIN CTAC, ex Hoechst Celanese. Another particularly preferred cationic surfactant for use in conditioners according to the invention is behenyltrimethylammonium chloride, available commercially, for example as GENAMIN KDMP, ex Clariant.

In conditioners for use in the invention, the level of other cationic conditioning surfactant is from 0.01 to 10%, more preferably 0.05 to 7.5%, most preferably 0.1 to 5% by total weight of cationic conditioning surfactant based on the total weight of the composition.

### Other components

### Silicones

Compositions according to the invention, will preferably also contain one or more silicone conditioning agents.

Particularly preferred silicone conditioning agents are silicone emulsions such as those formed from silicones such as polydiorganosiloxanes, in particular polydimethylsiloxanes which have the CTFA designation dimethicone, polydimethyl siloxanes having hydroxyl end groups which have the CTFA designation dimethiconol, and amino-functional polydimethyl siloxanes which have the CTFA designation amodimethicone.

The emulsion droplets may typically have a Sauter mean droplet diameter (D_{3,2}) in the composition of the invention ranging from 0.01 to 20 micrometer, more preferably from 0.2 to 10 micrometer. A suitable method for measuring the Sauter mean droplet diameter (D_{3,2}) is by laser light scattering using an instrument such as a Malvern Mastersizer.

Suitable silicone emulsions for use in compositions of the invention are available from suppliers of silicones such as Dow Corning and GE Silicones. The use of such pre-formed silicone emulsions is preferred for ease of processing and control of silicone particle size. Such pre-formed silicone emulsions will typically additionally comprise a suitable emulsifier such as an anionic or nonionic emulsifier, or mixture thereof, and may be prepared by a chemical emulsification process such as emulsion polymerisation, or by mechanical emulsification using a high shear mixer. Pre-formed silicone emulsions having a Sauter mean droplet diameter (D_{3,2}) of less than 0.15 micrometers are generally termed microemulsions.

Examples of suitable pre-formed silicone emulsions include emulsions DC2-1766, DC2-1784, DC-1785, DC-1786, DC-1788 and microemulsions DC2-1865 and DC2-1870, all available from Dow Corning. These are all emulsions/microemulsions of dimethiconol. Also suitable are amodimethicone emulsions such as DC2-8177 and DC939 (from Dow Corning) and SME253 (from GE Silicones).

Also suitable are silicone emulsions in which certain types of surface active block copolymers of a high molecular weight have been blended with the silicone emulsion droplets, as described for example in WO03/094874. In such materials, the silicone emulsion droplets are preferably formed from polydiorganosiloxanes such as those described above. One preferred form of the surface active block copolymer is according to the following formula:

HO(CH₂CH₂O)ₓ(CH(CH₃)CH₂O)_{y}(CH₂CH₂O)ₓ H

wherein the mean value of x is 4 or more and the mean value of y is 25 or more.

Another preferred form of the surface active block copolymer is according to the following formula:

(HO(CH₂CH₂O)ₐ(CH(CH₃)CH₂O)_{b})₂-N-CH₂-CH₂-N((OCH₂CH(CH₃))_{b}(OCH₂CH₂)ₐOH)₂

wherein the mean value of a is 2 or more and the mean value of b is 6 or more.

Mixtures of any of the above described silicone emulsions may also be used.

The above described silicone emulsions will generally be present in a composition of the invention at levels of from 0.05 to 10%, preferably 0.05 to 5%, more preferably from 0.5 to 2% by total weight of silicone based on the total weight of the composition.

### Further ingredients

The composition according to the invention may comprise any of a number of ingredients which are common to conditioning compositions

Other ingredients may include viscosity modifiers, preservatives, colouring agents, polyols such as glycerine and polypropylene glycol, chelating agents such as EDTA, antioxidants such as vitamin E acetate, fragrances, antimicrobials and sunscreens. Each of these ingredients will be present in an amount effective to accomplish its purpose. Generally these optional ingredients are included individually at a level of up to about 5% by weight of the total composition.

Preferably, compositions of this invention also contain adjuvants suitable for hair care. Generally such ingredients are included individually at a level of up to 2%, preferably up to 1%, by weight of the total composition.

Among suitable hair care adjuvants, are:
(i) natural hair root nutrients, such as amino acids and sugars. Examples of suitable amino acids include arginine, cysteine, glutamine, glutamic acid, isoleucine, leucine, methionine, serine and valine, and/or precursors and derivatives thereof. The amino acids may be added singly, in mixtures, or in the form of peptides, e.g. di- and tripeptides. The amino acids may also be added in the form of a protein hydrolysate, such as a keratin or collagen hydrolysate. Suitable sugars are glucose, dextrose and fructose. These may be added singly or in the form of, e.g. fruit extracts.
(ii) hair fibre benefit agents. Examples are:
   - ceramides, for moisturising the fibre and maintaining cuticle integrity. Ceramides are available by extraction from natural sources, or as synthetic ceramides and pseudoceramides. A preferred ceramide is Ceramide II, ex Quest. Mixtures of ceramides may also be suitable, such as Ceramides LS, ex Laboratoires Serobiologiques.
   - free fatty acids, for cuticle repair and damage prevention. Examples are branched chain fatty acids such as 18-methyleicosanoic acid and other homologues of this series, straight chain fatty acids such as stearic, myristic and palmitic acids, and unsaturated fatty acids such as oleic acid, linoleic acid, linolenic acid and arachidonic acid. A preferred fatty acid is oleic acid. The fatty acids may be added singly, as mixtures, or in the form of blends derived from extracts of, e.g. lanolin.

Mixtures of any of the above active ingredients may also be used.

In a second aspect there is provided a method for the manufacture of a conditioning composition according to the first aspect. The method comprising forming a conditioning gel phase which comprises a cationic surfactant and a fatty material and, separately forming a solution of the hydrophobically modified polymer, optionally with a cationic surfactant, which, if present, is added to the water first.

The two mixtures are then added to one another before the remaining ingredients are added to form the conditioning composition.

Preferably, the extra ingredients include perfumes, thickeners, preservatives, colours and conditioning silicones.

### Example 1: Composition 1 in accordance with the invention and Comparative Composition A

The following compositions were prepared:

**Table 1: Compositions of Comparative A and Composition 1 in accordance with the invention**

| **Material (based on 100 % active)** | **Amount (wt %)** | |
|---|---|---|
| | **Comparative A** | **Composition 1** |
| Behenyl Trimethyl Ammonium Chloride | 1.6 | 1.6 |
| Ceterearyl Alcohol | 3.2 | 3.2 |
| Amodimethicone/Dimethicone | 2.5 | 2.5 |
| Acrylates/Beheneth-25 Methacrylate Coploymer (Aculyn 28) | 0 | 0.1 |
| Fragrance/preservatives/water | to 100 | to 100 |

The conditioners A and 1 were prepared using the following method:
1. Water and copolymer were added to a suitable vessel and heated to 80 °C.
2. Cetearyl alcohol was then added.
3. At 80°C the Behenyl Trimethyl Ammonium Chloride (BTAC) was added and the resultant mixture mixed until opaque and thick.
4. The heat was then turned off and the quench water was added.
5. The mixture was then cooled to below 40°C the rest of the materials, including fragrance, were added.
6. Finally the formulation was mixed at high shear on a Silverson mixer at 5000 rpm for 5 minutes.

### Example 2: Treatment of hair with compositions A and 1

The hair used was dark brown European hair, in switches of 5 g weight and 6 inch length.

Hair was first treated with a cleansing shampoo using the following method:-
The hair fibres were held under running water for 30 seconds, shampoo applied at a dose of 0.1 ml of shampoo per 1g of hair and rubbed into the hair for 30 seconds. Excess lather was removed by holding under running water for 30 seconds and the shampoo stage repeated. The hair was rinsed under running water for 1 minute.

The wet hair was then treated with Conditioner A or 1 using the following method:-
Conditioner was applied to the wet hair at a dose of 0.2 ml of conditioner per 1g of hair and massaged into the hair for 1 minute. The hair was rinsed under running water for 1 minute and excess water removed.

### Example 3: Friction measurement of hair treated with Compositions A and 1

Friction was measured using the apparatus and method of the invention as follows: Friction was measured using a TA.XT2i Texture Analyser supplied by Stable Micro Systems, Surrey, UK, and a friction probe in the form of a stainless steel cylinder, which was coated with rubber material and fitted with a weight. The friction probe had surfactant on its outer (contact) surface. The load on the friction contact was approximately 138 g. When in use, an area of contact between the outer surface of the friction probe and the hair of approximately 1.0 cm² was achieved.

Surfactant was added to the probe as follows:
The probe was first washed with an aqueous composition of Sodium Lauryl Ether Sulphate (SLES) at a concentration of 14 wt %, by weight of the total aqueous surfactant composition, and rinsed with water. The probe was then soaked in a dilute solution of SLES having a concentration of 14 ppm, for 2 minutes, and then dried for 2 hours.

The methodology used to assess the friction properties of hair treated with Conditioners A and 1 was as follows:
A switch of hair was securely mounted onto the texture analyser, the hair fibres being aligned by combing before being secured in a flat configuration. The hair was immersed in the water bath. The friction probe was placed onto the hair and moved along the hair at a speed of 10 mms⁻¹ to measure the friction between the probe and the hair. The measurement was repeated 30 times.

The friction values reported below are of friction hysteresis in units of gfmm, obtained by integrating the total measured friction force over the total distance travelled by the probe, with and against cuticle.

The conditioner was pre-diluted at 20, 40 , 80 and 160 parts water and used to treat hair using the method described above. Friction measurements were then performed. Immersed friction measured on hair switches treated with Conditioners A and 1 are given in the Table below.

### Example 4: Friction measurement of hair treated with Compositions A and 1

**Table 2: Friction (gfmm) of hair treated with Comparative A and Composition 1 in accordance with the invention, after dilution levels of 20, 40, 80 and 160.**

| Dilution | Comparative A | Composition 1 | significance |
|---|---|---|---|
| 20 | 80.5 | 64.6 | ns |
| 40 | 81.4 | 100.6 | ns |
| 80 | 102.4 | 136.9 | >95% |
| 160 | 160 | 109.5 | ns |

It will be seen that a rinsed friction plateau is reached for hair treated with the composition of the invention sooner than with the comparative composition (at a dilution of 40 for composition 1 compared with 80 for composition A).

### Example 5: Composition 2 in accordance with the invention and Comparative Composition B

**Table 3: Compositions of Comparative B and Composition 2 in accordance with the invention**

| **Material (based on 100 % active)** | **Amount (wt %)** | |
|---|---|---|
| | **Comparative B** | **Composition 2** |
| Behenyl Trimethyl Ammonium Chloride | 2.3 | 2.3 |
| Ceterearyl Alcohol | 3.2 | 3.2 |
| Amodimethicone/Dimethicone | 1.4 | 1.4 |
| Acrylates/Beheneth-25 Methacrylate Coploymer (Aculyn 28) | 0 | 0.25 |
| Fragrance/preservatives/water | to 100 | to 100 |

The conditioners B and 2 were prepared using the method given in Example 1 above.

### Example 6: Viscosity of Compositions B and 2 under dilution

When a conditioning gel phase composition is applied to hair during a wash/care process, the gel phase is deposited onto the hair surface. When the deposited gel phase comes into contact with water (during a rinse step), the structure of the gel phase must be broken up in order for it to be efficiently removed from the hair. The greater the disruption to the gel phase, the easier and faster it is removed and, *ipso facto,* the less water is required to complete the rinse. The disruption to the composition gel phase can be indicated by a reduction in its viscosity upon dilution with water.

For any given quantity of water, the extent of viscosity reduction indicates how quickly and easily it will be removed from the hair. This correlates with the amount of water used to rinse a conditioning composition from hair.

In the following examples, viscosity measurements were carried out on aqueous dilutions of the neat composition (Compositions 1 and B).

Samples were measured using a Brookfield viscometer with a T-A spindle as well as RV5.

The samples were prepared as 150 g dilutions as follows:
Composition (for example 75 g for a 1 in 2 dilution) was added to a beaker. Water (75 g for a 1 in 2 dilution) was then added in small amounts with mixing until homogeneous.

The sample was left to equilibrate for one hour before measurement with the Brookfield viscometer.

In this way, a series of dilutions were prepared (ensuring consistent mixing and speed of water addition throughout).

The samples were measured using the Brookfield RVDV-II+ viscometer with the following conditions: T-A bar spindle: 0.5rpm; 60s measurement; 5 replicates per sample.

The results are given in the following table:

**Table 4: Viscosity of Composition 1 (in accordance with the invention) and Comparative Composition B.**

| | **Viscosity / cP** | | | **Normalised data** | |
|---|---|---|---|---|---|
| **Dilution** | **1** | **B** | | **1** | **B** |
| Neat | 517600 | 431200 | | 500000 | 500000 |
| 1 in 1.25 | 167000 | 286400 | | 161321.5 | 332096.5 |
| 1 in 1.5 | 99600 | 208000 | | 96213.29 | 241187.4 |
| 1 in 1.75 | 53200 | 143600 | | 51391.04 | 166512.1 |
| 1 in 2 | 25200 | 95600 | | 24343.12 | 110853.4 |
| 1 in 3 | 6000 | 27600 | | 5795.981 | 32003.71 |
| 1 in 4 | 400 | 7600 | | 386.3988 | 8812.616 |

It will be seen that the viscosity of the composition in accordance with the invention drops dramatically faster than that of the comparative example, ensuring saving of water during a rinse step of a conditioning process.

## Claims

1. A hair conditioner composition comprising
a) a conditioning gel phase comprising
i) from 0.01 to 10 wt %, by weight of the composition of a cationic surfactant,
ii) from 0.01 to 10 wt %, by weight of the composition of a fatty alcohol having from 8 to 22 carbon atoms;
b) from 0.01 to 5.0 wt %, by weight of the composition of a hydrophobically modified anionic polymer, comprising hydrophobic modification that comprises an alkyl group comprising from 6 to 30 carbon atoms; and
c) water
wherein the hair conditioner composition comprises from 0 to less than 0.1 weight %, by weight of the total composition of stearamidopropyldimethylamine.

2. Composition according to claim 1 wherein the polymer is an acrylate polymer.

3. Composition according to any preceding claim wherein the polymer is a methacrylate polymer.

4. Composition according to any preceding claim wherein the cationic surfactant has the formula N⁺(R¹)(R²)(R³)(R⁴), wherein R¹, R², R³ and R⁴ are independently (C₁ to C₃₀) alkyl or benzyl.

5. Composition according to claim 6 wherein one, two or three of R¹, R², R³ and R⁴ are independently (C₄ to C₃₀) alkyl and the other R¹, R², R³ and R⁴ group or groups are (C₁-C₆) alkyl or benzyl.

6. Composition according to any preceding claim wherein the cationic conditioning surfactant is selected from the group consisting of cetyltrimethylammonium chloride, behenyltrimethylammonium chloride and mixtures thereof.

7. Composition according to any preceding claim, which comprises from 0 to less than 0.1 wt % of each of stearamidopropyldimethylamine and stearamidopropyl dimethylamine stearate, by weight of the total composition.

8. A method of saving water during a rinse step of a conditioning process comprising the steps of applying to hair a composition comprising
a) a conditioning gel phase comprising
i) from 0.01 to 10 wt %, by weight of the composition of a cationic surfactant,
ii) from 0.01 to 10 wt %, by weight of the composition of a fatty alcohol having from 8 to 22 carbon atoms;
b) from 0.1 to 5.0 wt %, by weight of the composition of a hydrophobically modified anionic polymer comprising hydrophobic modification that comprises an alkyl group comprising from 12 to 30 carbon atoms; and
c) water
wherein the hair conditioner composition comprises from 0 to less than 0.1 weight %, by weight of the total composition of stearamidopropyldimethylamine compared with a comparative composition that differs from the composition in that it comprises no hydrophobically modified anionic polymer.

9. A method as claimed in claim 8 wherein the composition is as defined in any one of claims 2 to 7

10. A method as claimed in claimed in claim 9 wherein the hair is rinsed with water until the friction levels out to a plateau, as measured for example, using a Texture Analyser on hair that has been treated with the method of claim 8 or claim 9 wherein the composition has been pre-diluted with 20, 40 , 80 and 160 parts water

11. Use of a hydrophobically modified anionic polymer in a hair conditioner composition, as defined by any one of claims 1 to 7 to save water during a rinse step of a conditioning process compared with a comparative composition that differs from the composition in that it comprises no hydrophobically modified anionic polymer

12. Use as claimed in claim 11 to save water during a rinse step of a conditioning process until the friction levels out to a plateau, as measured for example, using a Texture Analyser on hair that has been treated with the method of claim 8 or claim 9 wherein the composition has been pre-diluted with 20, 40 , 80 and 160 parts water.

## Patentansprüche

1. Haarkonditionierende Zubereitung, umfassend
a) eine konditionierende Gelphase, umfassend
i) 0,01 bis 10 Gew.-% eines kationischen Tensids, bezogen auf das Gewicht der Zubereitung,
ii) 0,01 bis 10 Gew.-% eines Fettalkohols mit 8 bis 22 Kohlenstoffatomen, bezogen auf das Gewicht der Zubereitung;
b) 0,01 bis 5,0 Gew.-% eines hydrophob modifizierten anionischen Polymers, bezogen auf das Gewicht der Zubereitung, umfassend eine hydrophobe Modifikation, die eine Alkylgruppe mit 6 bis 30 Kohlenstoffatomen umfasst; und
c) Wasser,
wobei die haarkonditionierende Zubereitung 0 bis weniger als 0,1 Gewichts-% Stearamidopropyldimethylamin, bezogen auf das Gewicht der gesamten Zubereitung, umfasst.

2. Zubereitung nach Anspruch 1, wobei das Polymer ein Acrylatpolymer ist.

3. Zubereitung nach einem vorhergehenden Anspruch, wobei das Polymer ein Methacrylatpolymer ist.

4. Zubereitung nach einem vorhergehenden Anspruch, wobei das kationische Tensid die Formel N⁺(R¹)(R²)(R³)(R⁴) aufweist, worin R¹, R², R³ und R⁴, unabhängig voneinander, (C₁- bis C₃₀-)-Alkyl oder Benzyl sind.

5. Zubereitung nach Anspruch 6, wobei ein, zwei oder drei von R¹, R², R³ und R⁴, unabhängig voneinander, (C₄ bis C₃₀)-Alkyl ist/sind und die andere(n) R¹-, R²-, R³- und R⁴ -Gruppe oder -Gruppen (C₁-C₆)-Alkyl oder Benzyl ist/sind.

6. Zubereitung nach irgendeinem vorhergehenden Anspruch, wobei das konditionierende kationische Tensid aus der Gruppe ausgewählt ist, bestehend aus Cetyltrimethylammoniumchlorid, Behenyltrimethylammoniumchlorid und Mischungen davon.

7. Zubereitung nach einem vorhergehenden Anspruch, die 0 bis weniger als 0,1 Gew.-% jeweils von Stearamidopropyldimethylamin und Stearamidoproyldimethylaminstearat, bezogen auf das Gewicht der gesamten Zubereitung, umfasst.

8. Verfahren zum Einsparen von Wasser während eines Spülschritts eines Konditionierverfahrens, umfassend die Schritte des Auftragens einer Zubereitung auf das Haar, umfassend
a) eine konditionierende Gelphase, umfassend
i) 0,01 bis 10 Gew.-% eines kationischen Tensids, bezogen auf das Gewicht der Zubereitung,
ii) 0,01 bis 10 Gew.-% eines Fettalkohols mit 8 bis 22 Kohlenstoffatomen, bezogen auf das Gewicht der Zubereitung;
b) 0,1 bis 5,0 Gew.-% eines hydrophob modifizierten anionischen Polymers, bezogen auf das Gewicht der Zubereitung, umfassend eine hydrophobe Modifikation, die eine Alkylgruppe mit 12 bis 30 Kohlenstoffatomen umfasst; und
c) Wasser,
wobei die haarkonditionierende Zubereitung 0 bis weniger als 0,1 Gewichts-% Stearamidopropyldimethylamin, bezogen auf das Gewicht der gesamten Zubereitung, umfasst, wobei eine zum Vergleich herangezogene Vergleichszubereitung sich von der Zubereitung darin unterscheidet, dass sie kein hydrophob modifiziertes anionisches Polymer umfasst.

9. Verfahren nach Anspruch 8, wobei die Zubereitung nach einem der Ansprüche 2 bis 7 definiert ist.

10. Verfahren, wie im Anspruch 9 beansprucht, wobei das Haar mit Wasser gespült wird, bis sich die Reibung auf ein Plateau eingependelt hat, beispielsweise gemessen unter Verwendung eines Texturprüfgeräts am Haar, das nach dem Verfahren der Ansprüche 8 oder 9 behandelt wurde, wobei die Zubereitung mit 20, 40, 80 und 160 Teilen Wasser vorverdünnt wurde.

11. Verwendung eines hydrophob modifizierten anionischen Polymers in einer haarkonditionierenden Zubereitung, wie in irgendeinem der Ansprüche 1 bis 7 definiert, um während eines Spülschritts eines Konditionierverfahrens Wasser zu sparen, wobei eine zum Vergleich herangezogene Vergleichszubereitung sich von der Zubereitung darin unterscheidet, dass sie kein hydrophob modifiziertes anionisches Polymer umfasst.

12. Verwendung, wie im Anspruch 11 beansprucht, um während eines Spülschritts eines Konditionierverfahrens Wasser zu sparen, bis sich die Reibung auf ein Plateau eingependelt hat, beispielsweise gemessen unter Verwendung eines Texturprüfgeräts am Haar, das nach dem Verfahren der Ansprüche 8 oder 9 behandelt wurde, wobei die Zubereitung mit 20, 40, 80 und 160 Teilen Wasser vorverdünnt wurde.

## Revendications

1. Composition de conditionneur de cheveux comprenant
a) une phase de gel de conditionnement comprenant
i) de 0,01 à 10 % en masse, en masse de la composition d'un tensioactif cationique,
ii) de 0,01 à 10 % en masse, en masse de la composition d'un alcool gras ayant de 8 à 22 atomes de carbone ;
b) de 0,01 à 5,0 % en masse, en masse de la composition d'un polymère anionique hydrophobiquement modifié, comprenant une modification hydrophobe qui comprend un groupe alkyle comprenant de 6 à 30 atomes de carbone ; et
c) de l'eau
dans laquelle la composition de conditionneur de cheveux comprend de 0 à moins de 0,1 % en masse, en masse de la composition totale de stéaramidopropyldiméthylamine.

2. Composition selon la revendication 1, dans laquelle le polymère est un polymère d'acrylate.

3. Composition selon l'une quelconque des revendications précédentes, dans laquelle le polymère est un polymère de méthacrylate.

4. Composition selon l'une quelconque des revendications précédentes, dans laquelle le tensioactif cationique présente la formule N⁺(R¹)(R²)(R³)(R⁴), dans laquelle R¹, R², R³ et R⁴ sont indépendamment un alkyle en (C₁ à C₃₀) ou benzyle.

5. Composition selon la revendication 6, dans laquelle un, deux ou trois de R¹, R², R³ et R⁴ sont indépendamment un alkyle en (C₄ à C₃₀) et les autres groupe ou groupes R¹, R², R³ et R⁴ sont un alkyle en (C₁-C₆) ou benzyle.

6. Composition selon l'une quelconque des revendications précédentes, dans laquelle le tensioactif de conditionnement cationique est choisi dans le groupe consistant en chlorure de cétyltriméthylammonium, chlorure de béhényltriméthylammonium et mélanges de ceux-ci.

7. Composition selon l'une quelconque des revendications précédentes, qui comprend de 0 à moins de 0,1 % en masse de chacun de stéaramidopropyldiméthylamine et stéarate de stéaramidopropyldiméthylamine, en masse de la composition totale.

8. Procédé d'économie d'eau pendant une étape de rinçage d'un procédé de conditionnement comprenant les étapes d'application aux cheveux d'une composition comprenant
a) une phase de gel de conditionnement comprenant
i) de 0,01 à 10 % en masse, en masse de la composition d'un tensioactif cationique,
ii) de 0,01 à 10 % en masse, en masse de la composition d'un alcool gras ayant de 8 à 22 atomes de carbone ;
b) de 0,1 à 5,0 % en masse, en masse de la composition d'un polymère anionique hydrophobiquement modifié comprenant une modification hydrophobe qui comprend un groupe alkyle comprenant de 12 à 30 atomes de carbone ; et
c) de l'eau
dans lequel la composition de conditionneur de cheveu comprend de 0 à moins de 0,1 % en masse, en masse de la composition totale de stéaramidopropyldiméthylamine en comparaison avec une composition comparative qui est différente de la composition en ce qu'elle ne comprend aucun polymère anionique hydrophobiquement modifié.

9. Procédé selon la revendication 8, dans lequel la composition est comme définie dans l'une quelconque des revendications 2 à 7.

10. Procédé selon la revendication 9, dans lequel les cheveux sont rincés avec de l'eau jusqu'à ce que la friction se stabilise vers un plateau, comme mesuré par exemple, en utilisant un dispositif d'analyse de texture sur des cheveux qui ont été traités avec le procédé selon la revendication 8 ou revendication 9 dans lequel la composition a été pré-diluée avec 20, 40, 80 et 160 parties d'eau.

11. Utilisation d'un polymère anionique hydrophobiquement modifié dans une composition de conditionneur de cheveu, comme défini par l'une quelconque des revendications 1 à 7 pour économiser de l'eau pendant une étape de rinçage d'un procédé de conditionnement en comparaison avec une composition comparative qui est différente de la composition en ce qu'elle ne comprend aucun polymère anionique hydrophobiquement modifié.

12. Utilisation selon la revendication 11 pour économiser de l'eau pendant une étape de rinçage d'un procédé de conditionnement jusqu'à ce que la friction se stabilise vers un plateau, comme mesuré par exemple, en utilisant un dispositif d'analyse de texture sur des cheveux qui ont été traités avec le procédé selon la revendication 8 ou revendication 9 dans laquelle la composition a été pré-diluée avec 20, 40, 80 et 160 parties d'eau.
